# EUROPÄISCHE PATENTSCHRIFT

(11) **EP 2 740 365 B1**
(45) Veröffentlichungstag und Bekanntmachung des Hinweises auf die Patenterteilung: **18.04.2018**
(21) Anmeldenummer: 13186088.4
(22) Anmeldetag: 26.09.2013
(51) Int. Cl.: B07C 5/342, A22C 11/00, G01N 21/25, G01N 21/88, G01N 21/94, G01N 21/95, G01N 21/84, G01N 33/12

(54) **Verfahren und Vorrichtung zur Untersuchung eines in einem Pellvorgang zu pellenden Lebensmittelprodukts**
Method and device for examining a food product to be peeled in a peeling process
Procédé et dispositif d'examen d'un produit alimentaire à peler dans un processus de pelage

(30) Priorität: 05.12.2012 DE 102012222362; 13.12.2012 DE 102012223140
(43) Veröffentlichungstag der Anmeldung: 11.06.2014
(73) Patentinhaber: Weber Maschinenbau GmbH Breidenbach, 35236 Breidenbach (DE)
(72) Erfinder: Hallenberger, Jens, 35088 Battenberg (DE); Runkel, Andreas, 35216 Biedenkopf (DE)
(74) Vertreter: Manitz Finsterwald Patentanwälte PartmbB

(56) Entgegenhaltungen:
- EP-A1- 1 219 175
- EP-B1- 2 187 750
- US-B1- 7 460 227

## Beschreibung

Die vorliegende Erfindung betrifft ein Verfahren und eine Vorrichtung zur Untersuchung eines in einem Pellvorgang zu pellenden Lebensmittelprodukts daraufhin, ob die Pelle vollständig von dem Produkt entfernt wurde, wie beispielsweise aus der EP 1 219 175 A1 oder der US 7 460 227 B1 bekannt. Maschinen zum Pellen von Lebensmittelprodukten, wie etwa Würste, sind bekannt. Eine Pellmaschine ist zum Beispiel in der EP 2 187 750 B1 beschrieben. Bei einem in einer Pellmaschine durchgeführten Pellvorgang kann das Problem auftreten, dass die Pelle nicht vollständig von dem Produkt entfernt wird. Beispielsweise kann die Pelle reißen, während sie vom Produkt abgezogen wird, so dass der nicht abgezogene Teil der Pelle am Produkt verbleibt.

Zur Überprüfung, ob während eines Pellvorgangs die Pelle vollständig von einem Produkt entfernt wurde, ist es bekannt, Lichtschranken einzusetzen, um einen vom Produkt abgezogenen Teil der Pelle zu detektieren. Allerdings ist hieran nachteilig, dass ein am hinteren Ende des Produkts verbliebener Zipfel der Pelle nur schwer oder überhaupt nicht von den Lichtschranken detektierbar ist.

Außerdem ist bekannt, einen Metalldetektor zur Detektion eines am Zipfel der Pelle angebrachten Clips einzusetzen. Auf diese Weise kann allerdings nur ein metallischer Clip detektiert werden, wohingegen ein Clip aus Kunststoff nicht detektierbar ist. Außerdem sind derartige Metalldetektoren verhältnismäßig teuer.

Der vorliegenden Erfindung liegt die Aufgabe zugrunde, ein Verfahren beziehungsweise eine Vorrichtung zur Untersuchung eines Lebensmittelprodukts bereitzustellen, um mit verhältnismäßig geringem Aufwand und hoher Sicherheit feststellen zu können, ob Pelle an dem Produkt vorhanden ist.

Die Aufgabe wird durch ein Verfahren mit den Merkmalen des Anspruchs 1 beziehungsweise eine Vorrichtung mit den Merkmalen des Anspruchs 7 gelöst. Bevorzugte Weiterbildungen und Ausführungsformen der Erfindung sind in den abhängigen Ansprüchen angegeben.

Bei dem erfindungsgemäßen Verfahren zur Untersuchung eines in einem Pellvorgang zu pellenden Lebensmittelprodukts daraufhin, ob die Pelle vollständig von dem Produkt entfernt wurde, wird wenigstens ein Bild des Produkts, insbesondere eines hinteren Endes des Produkts, aufgenommen, und durch Auswerten des Bildes wird geprüft, ob zumindest ein Teil der Pelle noch am Produkt vorhanden ist.

Der vorliegenden Erfindung liegt der Gedanke zugrunde, durch Bildauswertung von wenigstens einem Bild des Produkts festzustellen, ob die Pelle vollständig von dem Produkt entfernt wurde. Das Verfahren kann insbesondere im Anschluss an einen Pellvorgang durchgeführt werden, um zu überprüfen, ob das Produkt während des Pellvorgangs vollständig gepellt wurde oder ob noch Pelle an dem Produkt verblieben ist. Mit dem Verfahren kann somit die ordnungsgemäße Durchführung des Pellvorgangs kontrolliert werden.

Bevorzugt erfolgt die Auswertung des Bildes daraufhin, ob ein Zipfel der Pelle am hinteren Ende des Produkts vorhanden ist oder nicht. Wird festgestellt, dass am Produkt kein Zipfel vorhanden ist, so wird angenommen, dass überhaupt keine Pelle am Produkt vorhanden ist. Überraschenderweise wurde festgestellt, dass in den allermeisten nicht ordnungsgemäß abgelaufenen Pellvorgängen der Zipfel am Produkt verblieb. Das Kriterium, ob der Zipfel am Produkt vorhanden ist, eignet sich daher besonders gut dazu, um eine Aussage dahingehend zu treffen, ob an dem Produkt zumindest ein Teil der Pelle vorhanden ist oder ob überhaupt keine Pelle am Produkt vorhanden ist.

Es kann aber auch vorkommen, dass der Zipfel vom Produkt entfernt wurde, aber trotzdem noch Pelle an wenigstens einer anderen Stelle am Produkt verblieben ist. Die noch verbliebene Pelle kann durch Auswerten des vom Produkt aufgenommenen Bildes erkannt werden. Dabei ist es vorteilhaft, wenn das gesamte Produkt aufgenommen wird. Besonders einfach kann insbesondere die Pelle, die am Produkt an einer anderen Stelle als am Zipfel verblieben ist, im Rahmen einer Bildauswertung detektiert werden, wenn das aufgenommene Bild im Hinblick auf eine oder mehrere Formen oder Konturen und/oder hinsichtlich der Helligkeitsverteilung und/oder hinsichtlich der Kontrastverteilung und/oder hinsichtlich der Farbverteilung ausgewertet wird, da auf diese Weise die Pelle einfach von dem Produkt und der Umgebung unterschieden und somit detektiert werden kann.

Die Prüfung wird anhand eines Vergleichs des aufgenommenen Bildes mit wenigstens einem Referenzbild eines gepellten Referenzprodukts durchgeführt. Das Referenzbild entspricht dabei einem in der jeweils gewünschten Weise gepellten, insbesondere vollständig von der Pelle befreiten, Produkt. Dabei kann das Referenzbild entweder nur wenigstens einen zum Überprüfen besonders geeigneten Teilbereich des Referenzprodukts in Form dessen hinteres Endes oder auch das gesamte Referenzprodukt zeigen. Vorzugsweise ist der im Referenzbild abgebildete Teilbereich des Referenzprodukts vollständig gepellt, da dann besonders einfach anhand des Vergleichs des Referenzbildes mit dem aufgenommenen Bild überprüft werden kann, ob noch Pelle am Produkt vorhanden ist. Durch Vergleichen des aufgenommenen Bildes mit dem Referenzbild kann insbesondere überprüft werden, ob das Ist-Bild, also das aufgenommene Bild, dem Soll-Bild, also dem Referenzbild, entspricht, um festzustellen, ob an dem Produkt zumindest ein Teil der Pelle vorhanden ist.

Die Erfindung betrifft ferner eine Vorrichtung zur Untersuchung eines in einem Pellvorgang zu pellenden Lebensmittelprodukts daraufhin, ob die Pelle vollständig von dem Produkt entfernt wurde, welche eine Bildaufnahmeeinrichtung zum Aufnehmen wenigstens eines Bildes des Produkts, insbesondere eines hinteren Endes des Produkts, und eine Bildauswerteeinrichtung aufweist, wobei die Bildauswerteeinrichtung dazu ausgebildet ist, durch Auswerten des Bildes zu prüfen, ob zumindest ein Teil der Pelle noch an dem Produkt vorhanden ist.

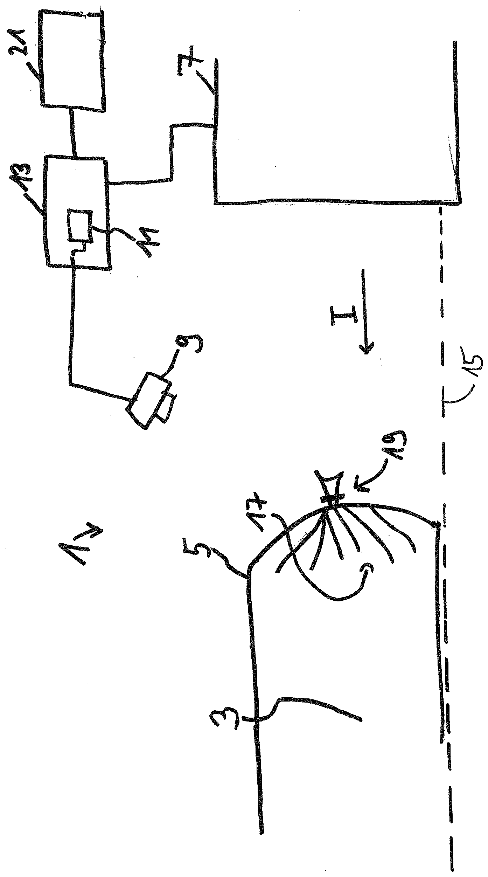

Die Erfindung wird nachfolgend beispielhaft mit Bezug auf die beiliegende Figur beschrieben, in der schematisch eine erfindungsgemäße Vorrichtung zur Untersuchung eines Lebensmittelprodukts dargestellt ist.

Die dargestellte Vorrichtung 1 ist zur Untersuchung eines Lebensmittelproduktes 3, wie etwa einer Wurst, vorgesehen, um festzustellen, ob eine an dem Produkt 3 ursprünglich vorhandene Pelle 5 in einem von einer Pellmaschine 7 durchgeführten Pellvorgang vollständig entfernt wurde. Die Vorrichtung 1 umfasst eine als Kamera ausgebildete Bildaufnahmeeinrichtung 9 und eine mit der Bildaufnahmeeinrichtung 9 gekoppelte Bildauswerteeinrichtung 11, die in eine mit der Pellmaschine 7 gekoppelten Steuerung 13 integriert ist.

Die Pellmaschine 7 kann beispielsweise entsprechend der in der EP 2 187 750 B1 beschriebenen Pellmaschine ausgebildet sein. Die Pellmaschine 7 übergibt das Produkt 3 im Anschluss an den Pellvorgang an eine Fördereinrichtung 15, wie etwa ein Förderband, die das Produkt 3 in einer Förderrichtung I fördert. Die Bildaufnahmeeinrichtung 9 ist oberhalb der Fördereinrichtung 15 angeordnet und kann das Produkt 3 erfassen, während es von der Fördereinrichtung 15 gefördert wird. Die Bildaufnahmeeinrichtung 9 nimmt wenigstens ein Bild des Produktes 3 auf und übermittelt das Bild an die Bildauswerteeinrichtung 11, die durch Auswerten des Bildes prüft, ob zumindest noch ein Teil der Pelle 5 am Produkt 3 vorhanden ist.

Insbesondere nimmt die Bildaufnahmeeinrichtung 9 wenigstens ein Bild ausschließlich des hinteren Endes 17 des Produkts 3 auf. Die Bildauswerteeinrichtung 11 überprüft dann durch Auswerten des Bildes, ob am hinteren Produktende 17 ein Zipfel 19 der Pelle 5 vorhanden ist. Wenn festgestellt wird, dass der Zipfel 19 nicht mehr vorhanden ist, dann wird angenommen, dass die Pelle 5 vollständig vom Produkt 3 entfernt wurde. Das Vorhandensein des Zipfels 19 dient somit als Entscheidungskriterium, ob generell am Produkt 3 noch Pelle 5 vorhanden ist oder ob das Produkt 3 vollständig gepellt wurde.

Die Bildauswertung daraufhin, ob der Zipfel 19 noch am Produkt 3 vorhanden ist, kann durchgeführt werden, indem das aufgenommene Bild mit wenigstens einem Referenzbild eines gepellten Produktes verglichen wird. Da, wie erwähnt, die Bildaufnahmeeinrichtung 9 insbesondere ein Bild ausschließlich des hinteren Endes 17 des Produkts 3 aufnimmt, ist im Referenzbild bevorzugt auch nur das gepellte hintere Ende des Referenzproduktes abgebildet.

Das Referenzbild kann dabei in der Bildauswerteeinrichtung 11 gespeichert sein. Erfindungsgemäß wird eine im Referenzbild vorhandene Kontur des hinteren Endes des Referenzprodukts mit einer im aufgenommenen Bild vorhandenen Kontur des hinteren Produktendes 17 verglichen, um festzustellen, ob die Konturen zumindest annähernd übereinstimmen. Falls eine innerhalb eines vorgegebenen Toleranzbereichs liegende Übereinstimmung der beiden Konturen festgestellt wird, wird angenommen, dass im aufgenommenen Bild kein Zipfel 19 abgebildet ist und somit das Produkt 3 vollständig gepellt wurde.

Zusätzlich kann auch hinsichtlich bestimmter Helligkeits-, Kontrast- und/oder Farbwerte verglichen bzw. nach bestimmten Helligkeits-, Kontrast- und/oder Farbmustern im aufgenommenen Bild gesucht werden.

Nicht dargestellt sind eine Vorrichtung zum Puffern und Aussortieren von fehlerhaft gepellten Produkten und eine Vorrichtung zum Aufschneiden von Lebensmitteln, die auch als Slicer bezeichnet wird, die in Förderrichtung I gesehen der Bildaufnahmeeinrichtung 9 nachgeordnet sein können. Die Steuerung 13 kann mit einer Steuerung 21 für den Slicer oder den Puffer, der auch als Beladepuffer bezeichnet wird, gekoppelt sein und an diese ein Signal ausgeben, wenn festgestellt wird, dass der Zipfel 19 am hinteren Produktende 17 noch vorhanden ist. Daraufhin wird das Produkt im Beladepuffer ausgeschleust, um es manuell nachzubearbeiten beziehungsweise nachzupellen und um zu vermeiden, dass das Produkt 3 aufgeschnitten wird und dabei der Zipfel 19 in den Bereich eines Schneidmessers des Slicers gelangt. Somit kann verhindert werden, dass ein am Zipfel 19 möglicherweise vorgesehener Metallclip das Schneidmesser beschädigt.

Bei dem vorstehend beschriebenen Beispiel handelt es sich bei der Bildaufnahmeeinrichtung 9 um eine Kamera. Alternativ kann die Bildaufnahmeeinrichtung 9 als ein- oder mehrzeiliger Bildsensor ausgestaltet sein und z.B. einen oder mehrere CCD-Sensoren umfassen.

### Bezugszeichenliste

- 1: Vorrichtung
- 3: Lebensmittelprodukt
- 5: Pelle
- 7: Pellmaschine
- 9: Bildaufnahmeeinrichtung
- 11: Bildauswerteeinrichtung
- 13: Steuerung
- 15: Fördereinrichtung
- 17: hinteres Ende
- 19: Zipfel
- 21: Beladepuffersteuerung oder Slicersteuerung

- I: Förderrichtung

## Patentansprüche

1. Verfahren zur Untersuchung eines in einem Pellvorgang zu pellenden Lebensmittelprodukts (3) daraufhin, ob die Pelle (5) vollständig von dem Produkt (3) entfernt wurde, bei dem
wenigstens ein Bild des Produkts (3), insbesondere eines hinteren Endes (17) des Produkts (3), aufgenommen wird, und
durch Auswerten des Bildes geprüft wird, ob zumindest ein Teil der Pelle (5) am Produkt (3) vorhanden ist;
**dadurch gekennzeichnet, dass**
die Prüfung anhand eines Vergleichs des aufgenommenen Bildes mit wenigstens einem Referenzbild zumindest eines gepellten Teilbereichs eines Referenzprodukts durchgeführt wird, wobei eine im Referenzbild vorhandene Kontur des hinteren Endes des Referenzprodukts mit einer im aufgenommenen Bild vorhandenen Kontur des hinteren Endes (17) des Produkts (3) verglichen wird, und bei zumindest innerhalb einer vorgegebenen Toleranz festgestellten Übereinstimmung der beiden Konturen angenommen wird, dass die Pelle (5) vollständig von dem Produkt (3) entfernt wurde.

2. Verfahren nach Anspruch 1,
**dadurch gekennzeichnet, dass**
das Bild erst nach Abschluss des Pellvorgangs aufgenommen wird.

3. Verfahren nach Anspruch 1 oder 2,
**dadurch gekennzeichnet, dass**
ausschließlich das hintere Produktende (17) aufgenommen wird.

4. Verfahren nach einem der vorhergehenden Ansprüche,
**dadurch gekennzeichnet, dass**
zumindest ein Teil des Bildes hinsichtlich einer oder mehrerer Formen oder Konturen, hinsichtlich der Helligkeitsverteilung und/oder hinsichtlich der Kontrastverteilung und/oder hinsichtlich der Farbverteilung ausgewertet wird.

5. Verfahren nach einem der vorhergehenden Ansprüche,
**dadurch gekennzeichnet, dass**
das Bild daraufhin ausgewertet wird, ob ein Zipfel (19) der Pelle (5) am hinteren Ende (17) des Produkts (3) vorhanden ist, und dass angenommen wird, dass überhaupt keine Pelle (5) am Produkt (3) vorhanden ist, wenn festgestellt wird, dass kein Zipfel (19) vorhanden ist.

6. Verfahren nach einem der vorhergehenden Ansprüche,
**dadurch gekennzeichnet, dass**
die Prüfung anhand eines Vergleichs des aufgenommenen Bildes mit wenigstens einem Referenzbild zumindest eines hinteren Endes eines Referenzprodukts durchgeführt wird.

7. Vorrichtung (1) zur Untersuchung eines in einem Pellvorgang zu pellenden Lebensmittelprodukts (3) daraufhin, ob die Pelle (5) vollständig von dem Produkt entfernt wurde,
mit einer Bildaufnahmeeinrichtung (9) zum Aufnehmen wenigstens eines Bildes des Produkts (3), insbesondere eines hinteren Endes (17) des Produkts (3), und einer Bildauswerteeinrichtung (11), die dazu ausgebildet ist, durch Auswerten des Bildes zu prüfen, ob zumindest ein Teil der Pelle (5) noch an dem Produkt (3) vorhanden ist;
**dadurch gekennzeichnet, dass**
die Prüfung anhand eines Vergleichs des aufgenommenen Bildes mit wenigstens einem Referenzbild zumindest eines gepellten Teilbereichs eines Referenzprodukts durchgeführt wird, wobei eine im Referenzbild vorhandene Kontur des hinteren Endes des Referenzprodukts mit einer im aufgenommenen Bild vorhandenen Kontur des hinteren Endes (17) des Produkts (3) verglichen wird, und bei zumindest innerhalb einer vorgegebenen Toleranz festgestellten Übereinstimmung der beiden Konturen angenommen wird, dass die Pelle (5) vollständig von dem Produkt (3) entfernt wurde.

8. Vorrichtung nach Anspruch 7,
**dadurch gekennzeichnet, dass**
die Bildaufnahmeeinrichtung (9) wenigstens einen Bildsensor, insbesondere eine Kamera, umfasst.

9. Vorrichtung nach Anspruch 7 oder 8,
**dadurch gekennzeichnet, dass**
eine Pellmaschine (7) zur Durchführung des Pellvorgangs vorgesehen ist, wobei die Bildaufnahmeeinrichtung (9) in Förderrichtung (I) des Produkts (3) gesehen der Pellmaschine (7) und/oder dem Pellvorgang oder Pellprozess nachgeordnet ist.

## Claims

1. A method of examining a food product (3) to be skinned in a skinning procedure as to whether the skin (5) was completely removed from the product (3), in which
at least one image of the product (3), in particular of a rear end (17) of the product (3), is recorded; and
a check is made by evaluating the image whether at least one part of the skin (5) is present at the product (3),
**characterized in that**
the check is carried out by means of a comparison of the recorded image with at least one reference image of at least one skinned part region of a reference product, with a contour of the rear end of the reference product present in the reference image being compared with a contour of the rear end (17) of the product (3) present in the recorded image, and with it being assumed on agreement of the two contours at least within a predefined tolerance that the skin (5) was completely removed from the product (3).

2. A method in accordance with claim 1,
**characterized in that**
the image is only recorded after the completion of the skinning procedure.

3. A method in accordance with claim 1 or claim 2,
**characterized in that**
only the rear product end (17) is recorded.

4. A method in accordance with any one of the preceding claims,
**characterized in that**
at least one part of the image is evaluated with respect to one or more shapes or contours, with respect to the brightness distribution and/or with respect to the contrast distribution and/or with respect to the color distribution.

5. A method in accordance with any one of the preceding claims,
**characterized in that**
the image is then evaluated as to whether a point (19) of the skin (5) is present at the rear end (17) of the product (3); and **in that** it is assumed that no skin (5) at all is present at the product (3) when it is determined that no point (19) is present.

6. A method in accordance with any one of the preceding claims,
**characterized in that**
the check is carried out by means of a comparison of the recorded image with at least one reference image of at least one rear end of a reference product.

7. An apparatus (1) for examining a food product (3) to be skinned in a skinning procedure as to whether the skin (5) was completely removed from the product,
comprising an image recording device (9) for recording at least one image of the product (3), in particular a rear end (17) of the product (3), and an image evaluation device (11) which is configured to check by evaluating the image whether at least one part of the skin (5) is still present at the product (3),
**characterized in that**
the check is carried out by means of a comparison of the recorded image with at least one reference image of at least one skinned part region of a reference product, with a contour of the rear end of the reference product present in the reference image being compared with a contour of the rear end (17) of the product (3) present in the recorded image, and with it being assumed on agreement of the two contours at least within a predefined tolerance that the skin (5) was completely removed from the product (3).

8. An apparatus in accordance with claim 7,
**characterized in that**
the image recording device (9) comprises at least one image sensor, in particular a camera.

9. An apparatus in accordance with claim 7 or claim 8,
**characterized in that**
a skinning machine (7) is provided for carrying out the skinning procedure, with the image recording device (9) being arranged downstream of the skinning machine (7) and/or of the skinning procedure or of the skinning process viewed in the conveying direction (I) of the product (3).

## Revendications

1. Procédé pour examiner un produit alimentaire (3) à peler dans une opération de pelage, pour savoir si la peau (5) a été entièrement enlevée du produit (3), dans lequel
au moins une image du produit (3), en particulier d'une extrémité arrière (17) du produit (3), est prise, et
par évaluation de l'image, il est contrôlé si une partie au moins de la peau (5) est présente sur le produit (3) ;
**caractérisé en ce que**
le contrôle est effectué en se basant sur une comparaison de l'image prise avec au moins une image de référence d'au moins une zone partielle pelée d'un produit de référence, un contour de l'extrémité arrière du produit de référence, présent dans l'image de référence, étant comparé avec un contour de l'extrémité arrière (17) du produit (3), présent dans l'image prise, et lors d'une concordance des deux contours constatée au moins à l'intérieur d'une tolérance prédéterminée, il est supposé que la peau (5) a été entièrement enlevée du produit (3).

2. Procédé selon la revendication 1,
**caractérisé en ce que**
l'image n'est prise qu'après terminaison de l'opération de pelage.

3. Procédé selon la revendication 1 ou 2,
**caractérisé en ce que**
exclusivement l'extrémité arrière (17) du produit est prise.

4. Procédé selon l'une des revendications précédentes,
**caractérisé en ce que**
une partie au moins de l'image est évaluée à l'égard d'une ou de plusieurs formes ou d'un ou de plusieurs contours, à l'égard de la répartition de la luminosité et/ou à l'égard de la répartition des contrastes et/ou à l'égard de la répartition des couleurs.

5. Procédé selon l'une des revendications précédentes,
**caractérisé en ce que**
l'image est évaluée pour savoir si un bout (19) de la peau (5) est présent à l'extrémité arrière (17) du produit (3), et
**en ce qu'**il est supposé qu'il n'y a pas du tout de peau (5) sur le produit (3) lorsqu'il est constaté qu'aucun bout (19) n'est présent.

6. Procédé selon l'une des revendications précédentes,
**caractérisé en ce que**
l'examen est effectué en se basant sur une comparaison de l'image prise avec au moins une image de référence d'une extrémité arrière au moins d'un produit de référence.

7. Dispositif (1) pour examiner un produit alimentaire (3) à peler dans une opération de pelage pour savoir si la peau (5) a été entièrement enlevée du produit,
comportant un moyen de prise d'image (9) pour prendre au moins une image du produit (3), en particulier d'une extrémité arrière (17) du produit (3), et un moyen d'évaluation d'image (11) qui est réalisé pour contrôler par évaluation de l'image, si une partie au moins de la peau (5) est encore présente sur le produit (3),
**caractérisé en ce que**
le contrôle est effectué en se basant sur une comparaison de l'image prise avec au moins une image de référence d'au moins une zone partielle pelée d'un produit de référence, un contour de l'extrémité arrière du produit de référence, présent dans l'image de référence, étant comparé avec un contour de l'extrémité arrière (17) du produit (3), présent dans l'image prise, et lors d'une concordance des deux contours constatée au moins à l'intérieur d'une tolérance prédéterminée, il est supposé que la peau (5) a été entièrement enlevée du produit (3).

8. Dispositif selon la revendication 7,
**caractérisé en ce que**
le moyen de prise d'image (9) comprend au moins un capteur d'image, en particulier une caméra.

9. Dispositif selon la revendication 7 ou 8,
**caractérisé en ce que**
il est prévu une machine à peler (7) pour mettre en oeuvre l'opération de pelage, le moyen de prise d'image (9) étant agencé en aval de la machine à peler (7) et/ou de l'opération de pelage ou du processus de pelage, vu en direction de convoyage (I) du produit (3).
